# EUROPÄISCHE PATENTSCHRIFT

(11) **EP 1 785 107 B1**
(45) Veröffentlichungstag und Bekanntmachung des Hinweises auf die Patenterteilung: **31.12.2008**
(21) Anmeldenummer: 06021560.5
(22) Anmeldetag: 13.10.2006
(51) Int. Cl.: A61F 2/84, A61F 2/90, A61F 2/06

(54) **Applikationssystem für einen Stent**
Stent delivery system
Système de pose de stent

(30) Priorität: 09.11.2005 DE 102005053393
(43) Veröffentlichungstag der Anmeldung: 16.05.2007
(73) Patentinhaber: BIOTRONIK VI Patent AG, 6341 Baar (CH)
(72) Erfinder: Delaloye, Stéphane, Dr., 8180 Bülach (CH); Koop, Karsten, 18057 Rostock (DE); Pantic, Dragica, 8051 Zürich (CH); Rast, Barbara, 8165 Oberwenigen (CH)
(74) Vertreter: Lindner-Vogt, Karin L.

(56) Entgegenhaltungen:
- US-A- 5 776 141
- US-A- 5 935 135
- US-A1- 2003 074 044
- US-B1- 6 494 906

## Beschreibung

Die Erfindung betrifft ein Applikationssystem für einen Stent.

In stetig zunehmendem Umfang werden in der modernen Implantationstechnologie Stents zur Stabilisierung/Abstützung von Hohlorganen genutzt. Der Ansatz scheint insbesondere geeignet für koronare Herzerkrankungen wie akute Myokardinfarkte, die eine der häufigsten Todesursachen in Westeuropa und Amerika darstellen. In mehr als 80% der Fälle ist die Ursache des Myokardinfarktes der thrombotische Verschluss einer Koronararterie durch Ruptur einer atheromatösen Plaque bei vorbestehender stenosierender Atheromatose. Entscheidende Faktoren für die Langzeitprognose nach akutem Myokardinfarkt sind (i) eine effektive und langanhaltende Wiedereröffnung der Infarktarterie, (ii) eine Dauer des thrombotischen Gefäßverschlusses, (iii) die Verhinderung eines größeren Myokardverlustes und eines ventrikulären Remodeling und (iv) die Beherrschung rhythmogener Komplikationen. Die genannten Faktoren bestimmen nicht nur die kardiovaskuläre Mortalität, sondern auch die Lebensqualität nach dem Infarkt.

Seit mehr als 20 Jahren sind nicht-operative Methoden zur Stenose-Behandlung etabliert, bei denen unter anderem durch Ballondilatation (PTA Perkutane transluminale Angioplastie) das verengte oder verschlossene Blutgefäß wieder aufgeweitet wird. Dieses Vorgehen hat sich insbesondere bei der Therapie des akuten Myokardinfarktes bewehrt. Zur Verhinderung eines erneuten Verschlusses des geweiteten Gefäßes durch Obstruktion wird ein Stent eingesetzt.

Zum Einbringen und ortgenauen Platzieren des Stents - im Weiteren zusammenfassend als Applizieren bezeichnet - bedarf es eines Applikationssystems.

Das System umfasst zwei Komponenten: Das Implantat selbst und einen Katheter, mit dem das Applizieren des Stents durchführbar ist. Beide Komponenten müssten aufeinander abgestimmt sein, um das Applizieren des Stents möglichst zuverlässig und mit minimal-operativem Aufwand durchzuführen.

Auf Seiten des Stents gilt es, eine Struktur zur Verfügung zu stellen, die einerseits den Implantationsvorgang unterstützt und andererseits sicherstellt, dass am Implantationsort die angestrebte Funktionalität wahrgenommen werden kann. Dies wird unter anderem dadurch erreicht, dass der rohrförmige Stent expandierbar ausgelegt ist; beim Applizieren wird der Stent zunächst in einem nicht-expandierten Zustand an den Implantationsort verbracht und dort mit geeigneten Mitteln in einen expandierten Zustand überführt. Das Expandieren kann dabei durch Beaufschlagen mit einer mechanischen Kraft, z. B. Inflation eines Ballons, bewirkt werden oder aus der Struktur selbst resultieren, wie etwa beim Einsatz von Gedächtnismaterialien im Stent.

Auf Seiten des Katheters sind Anpassungen u. a. in Abhängigkeit vom eingesetzten Stenttyp notwendig: man unterscheidet zwischen selbstexpandierenden Stents und ballonexpandierbaren Stents. Für den erstgenannten Stenttyp müssen Mittel vorgesehen sein, die die Selbstexpansion des Stents induzieren. Der letztgenannte Stenttyp macht den Einsatz eines Ballonkatheters notwendig, und durch Inflation des Ballons wird der Stent am Implantationsort aufgeweitet.

Ein Teilproblem bei der Optimierung derartiger Applikationssysteme für Stents besteht darin, ein Verrutschen oder gar den Verlust des Stents während dem Transport zum Implantationsort und während der Implantation zu verhindern. Ein Ansatzpunkt sieht vor, den auf den Katheter aufgesetzten Stent mit einer den Stent ganz oder in Teilen überdeckenden Schutzfolie zu überziehen. Die Folie soll dabei in der Regel so beschaffen sein, dass sie entweder beim Expandieren des Stents reißt oder sich zusammen mit dem Stent elastisch dehnen lässt. Der Ansatz wird unweigerlich dazu führen, dass Teile oder die gesamte Folie im Körper verbleibt und dass ein mechanischer Widerstand der Folie das Expansionsverhalten des Stents beeinflusst. Weiterhin ist ein Umfang des Applikationssystems vergrößert.

Weitere Beispiele für solche Befestigungssysteme sind aus der US 5,776,141 bekannt, bei der kurze Bänder einen Stent auf einem "passiv" dilatierbaren Abschnitt eines Katheters halten. "Passiv" im Sinne der Anmeldung bedeutet, dass der dilatierbare Abschnitt so hergerichtet ist, dass er radial expandieren kann, indem ein separater Ballonkatheter in das Lumen dieses Katheters eingeführt wird und durch Dilatieren des Katheterballons_diesen "passiv" dilatierbaren Abschnitt expandiert.

Weiter ist aus der US 6,494,906 bekannt, dass ein Stent mit einem Halteelement auf dem Ballon eines Ballonkatheters platziert werden kann und gegen Verrutschen dadurch gesichert ist, dass das Halteelement mit einer Ballonfalte zusammenwirkt.

Aufgabe der vorliegenden Erfindung ist es, ein verbessertes, zumindest aber alternatives Applikationssystem für einen Stent bereitzustellen, das Schutz vor Verlust oder dem Verrücken des Stents auf dem Katheter während dem Transport zum Implantationsort und während der Implantation bietet.

Diese Aufgabe wird durch das Applikationssystem für einen Stent gemäß Anspruch 1 gelöst. Erfindungsgemäß umfasst das Applikationssystem einen Ballonkatheter mit zumindest einer mit dem Katheter fest verbundenen Haltestruktur und einen auf den Katheter gezogenen Stent mit wenigstens einem Halteelement. Die Haltestruktur stellt einen Haltebereich bereit, der zur Aufnahme und Festlegung des Halteelementes ausgebildet ist. Das Halteelement des Stents und die Haltestruktur des Katheters sind so in Geometrie und Lage aufeinander abgestimmt, dass
(i) sich das Halteelement im nicht-expandierten Zustand des Stents im Haltebereich der Haltestruktur befindet und
(ii) die Haltestruktur und/oder das Halteelement beim Expandieren des Stents oder zumindest im expandierten Zustand des Stents so zueinander versetzt sind, dass sich das Halteelement nicht mehr im Haltebereich der Haltestruktur befindet.

Als Haltestruktur sind zwei kurze Bänder vorgesehen, deren Enden in dem Bereich des Ballons befestigt sind, der den Stent trägt, oder direkt durch den Stent auf dem Ballon fixiert werden und die Enden jeweils über eine Verbindungsstelle an den Katheter gebunden sind.

Mit Hilfe des erfindungsgemäßen Applikationssystems ist es möglich, dem Stent auf dem Katheter Halt zu verleihen, ohne dass diese Strukturen beim Applizieren stören oder diese Strukturen ganz oder in Teilen im Körper des Patienten verbleiben. Das erfindungsgemäße Applikationssystem erhöht die Gleithaltekraft des Stents auf dem Katheter durch die Fixierung in axialer Richtung und verhindert ein unerwünschtes Öffnen des Stents in kurvigen Gefäßen durch die Fixierung des Stents in radialer Richtung.

Die Verbindung zwischen Band und Katheter an den Verbindungsstellen kann beispielsweise durch Schweißen oder Kleben erfolgen. Vorzugsweise kann das Band über den Ballon des Katheters gespannt sein oder das Band ist mit seinem einen Ende in dem Abschnitt des Ballons, der den Stent aufnimmt, und mit dem anderen Ende außerhalb dieses Bereiches am Katheter befestigt. Ein Haltebereich der Haltestruktur im Sinne der Erfindung erstreckt sich zwischen der Katheteroberfläche und der Unterseite des Bandes. Das komplementäre Halteelement am Stent wird im nicht-expandierten Zustand des Stents unter das Band geschoben.

Der Katheter zeichnet sich demnach dadurch aus, dass er eine Haltestruktur aufweist, die fest mit ihm verbunden ist, z. B. durch Kleben oder Schweißen, oder durch einstückige Ausführung zusammen mit einem weiteren Bauteil des Katheters, beispielsweise dem Ballon oder dem Katheterschaft.

Ein Haltebereich der Haltestruktur ist erfindungsgemäß zur Aufnahme und Festlegung des Halteelementes ausgebildet. Dies bedeutet, dass der Haltebereich in seiner Geometrie als auch in den Eigenschaften der zur Realisation dieses Haltebereichs notwendigen Bauelemente der Halterstruktur an das Halteelement des Stents anzupassen ist. Eine geometrische Anpassung in diesem Sinne verlangt, dass der Haltebereich zumindest derart in seinen Abmessungen vorzugeben ist, dass er das Halteelement aufnehmen kann. Sind die Bauelemente zur Definition des Haltebereichs beweglich, insbesondere durch Verwendung elastomerer Werkstoffe, so wird dies bei der Auslegung der Haltestruktur zu berücksichtigen sein. Eine spezifische Haltestruktur wird sich der Fachmann in Kenntnis des komplementären Halteelementes mit geringem Aufwand erarbeiten. Der Fachmann kann sich dabei auch an den Beispielen weiter unten orientieren.

Das Halteelement des Stents und die Haltestruktur des Katheters sind erfindungsgemäß in Geometrie und Lage in besonderer Weise aufeinander abgestimmt. Im nicht-expandierten Zustand befindet sich das Halteelement des Stents im Haltebereich der Haltestruktur und kann dort unterstützt durch z. B. Klemm- und Reibkräfte festgelegt sein. Auf diese Weise wird sichergestellt, dass der Stent sich nicht während des Führens des Katheters zum Implantationsort auf dem Katheter verschiebt oder gar verloren geht. Am Implantationsort wird der Stent dann aufgeweitet. Die Expansion des Stents beinhaltet einen für die Erfindung wesentlichen Aspekt: Die Strukturelemente des Stents werden bei der Expansion teils verformt. Dies bedingt wiederum, dass eine Ausgangslage eines spezifischen Strukturelementes in Bezug auf weitere Strukturelemente des Stents von einer relativen Lage dieses Strukturelementes nach Expansion abweichen kann. Mit anderen Worten, eine Bewegung einzelner Strukturelemente in der (sich bei Expansion vergrößernden) Umlauffläche des Stents findet statt. Strukturelemente, die sich in diesem Sinne in der Umlauffläche des Stents bewegen, sind als Halteelemente für die erfindungsgemäßen Zwecke geeignet. Der Fachmann wird das Ausmaß der Bewegung der Strukturelemente bei der Expansion des Stents entweder durch Modelle erfassen oder durch einfache Expansionsversuche ermitteln können. Strukturelemente mit einer relativ großen Bewegung bei der Expansion eignen sich besonders für die erfindungsgemäßen Zwecke.

Mit Kenntnis der Bewegung des als Halteelement ausgewählten Strukturelementes wird der Fachmann eine Auslegung der komplementären Haltestruktur auf dem Katheter vornehmen müssen. Der Aufnahmebereich der Haltestruktur ist dabei so vorzugeben, dass die Haltestruktur und/oder das Halteelement beim Expandieren des Stents oder zumindest im expandierten Zustand des Stents zueinander versetzt wird, und zwar in dem Umfang, dass sich das Halteelement nicht mehr im Haltebereich der Haltestruktur befindet. Mit anderen Worten, der Stent wird nicht mehr durch die Haltestruktur des Katheters fixiert. Der Fachmann wird anhand dieser einfachen Überlegungen entweder ausgehend von der Haltestruktur des Katheters oder ausgehend vom Halteelement des Stents die notwendigen Anpassungen in Gestalt und Lage des jeweils komplementären Elementes festlegen können. Der Fachmann kann sich dabei auch an den Beispielen weiter unten orientieren.

Ein für die Zwecke der Erfindung geeigneter Stent hat zumindest ein Halteelement der zuvor beschriebenen Art. Der Stent weist üblicherweise ein Grundgerüst aus Stützstreben auf, deren relative Anordnung und Dimensionierung im hohen Maße variabel ist. Vorzugsweise besteht das Grundgerüst des Stents aus einem metallischen Material, da die für die erfindungsgemäßen Zwecke notwendige Bewegung einzelner Strukturelemente bei Vorgabe eines derartigen Werkstoffs definierter als zum Beispiel bei polymeren Werkstoffen abläuft und so die Umsetzung der erfindungsgemäßen Fixierung erleichtert. Besonders bevorzugt ist das Material eine gegebenenfalls auch biodegradierbare Magnesiumlegierung oder ein Material mit vergleichbarem E-Modul.

Das Applikationssystem kann mehrere Haltestrukturen und Halteelemente umfassen. Vorzugsweise weist der Katheter 3 bis 10 Haltestrukturen auf, wobei vorzugsweise 3 bis 5 Haltestrukturen gleichmäßig um den Umfang des Katheters angeordnet sind. Der Stent weist eine der Anzahl der Haltestrukturen des Katheters entsprechende Anzahl von Halteelementen auf. Die Haltestrukturen auf dem Katheter können sich über die gesamte Länge des Bereiches erstrecken, der den Stent aufnimmt, oder sie sind lediglich im Bereich der Stentenden vorhanden.

Gerade das Zusammenspiel zwischen dem Ballon und der obig näher erläuterten Bewegung der Strukturelemente des Stents auf dem Ballon bietet sich für das erfindungsgemäße Applikationssystem an.

Nach einer bevorzugten Ausführungsform der vorgenannten Ausführungsvariante besteht das Band aus einem thermoplastischen Elastomer. Dies hat zum einen den Vorteil, dass die Elastizität des Materials zur Erhöhung der Haltekraft nutzbar ist, also das Halteelement unter ein gespanntes Band geschoben wird und durch die entstehende Spannkraft die Haltekraft erhöht wird. Weiterhin hat das Material den Vorteil, dass ein Reißen des Bandes während der Expansion des Stents vermieden werden kann und bei der anschließenden Deflation des Ballons die Haltestruktur weitgehend in ihre Ausgangslage rückführbar ist.

Vorzugsweise bestehen bei vorgenannten Ausführungsformen das Band und der Katheter im Bereich der Verbindungsstelle aus dem gleichen Material. Hierdurch kann eine besonders einfache, aber auch zuverlässige Anbindung der Haltestruktur, z. B. durch Schweißverfahren oder Klebung erfolgen; da die Materialeigenschaften der beiden zu verbindenden Komponenten gleich sind, wird ein Abreißen der Haltestruktur bei mechanischer Beanspruchung vermieden.

Die Haltestruktur kann im Haltebereich eine Außenkante aufweisen. Diese Außenkante steht vorzugsweise zur Längsachse des Katheters in einem Winkel von weniger als 45°, insbesondere weniger als 20°, besonders bevorzugt weniger als 10°. Mit den genannten Winkelvorgaben ist sichergestellt, dass sich die Haltestruktur beim Ein- und Ausführen des Katheters in den Körper des Patienten nicht verhakt.

Vorzugsweise ist weiterhin das Halteelement des Stents in Form einer offenen Lasche, die vorzugsweise am Stentende angeordnet ist, ausgeführt. Unter offener Lasche im Sinne der Erfindung wird ein Halteelement verstanden, das ein Stegelement umfasst, welches an einem Ende mit den weiteren Strukturelementen des Stents verbunden ist, aber an seinem anderen Ende frei von jedweder weiteren Anbindung an die Stentstruktur ist. Eine Ausrichtung dieses Stegelementes, das die offene Lasche bildet, kann dabei in Umfangsrichtung, aber gegebenenfalls auch in Längsrichtung oder einem dazwischen liegenden Winkel erfolgen, je nachdem, wie die dazugehörige, komplementäre Haltestruktur des Katheters ausgebildet ist bzw. angeordnet ist. Bevorzugt sind Stegelemente für offene Laschen, die mit ihren freien Enden in Umlaufrichtung weisen. Stents mit einem Halteelement im vorgenannten Sinne sind aus dem Stand der Technik nicht bekannt und daher ein weiterer Gegenstand der vorliegenden Erfindung.

Eine weitere Ausführungsform des Applikationssystems sieht vor, dass der Stent eine Strebe aufweist, die im gecrimpten Zustand zumindest einen Abschnitt mit zickzack- oder wellenförmigem Verlauf aufweist, der Abschnitt als Halteelement dient, und der Abschnitt so ausgebildet ist, dass er beim Expandieren des Stents relativ zur Haltestruktur des Katheters versetzt wird. Bei einem solchen Halteelement wird das geometrisch bedingte Öffnen der Struktur im Zuge der Expansion des Stents zum Freisetzen aus der Haltestruktur des Katheters genutzt. Vorzugsweise ist der Abschnitt, der als Haltelement dient, Teil einer ringförmig umlaufenden Strebe des Stents, die vorzugsweise am Stentende des Stents angeordnet ist. Derartige Strukturen lassen sich fertigungstechnisch besonders einfach realisieren.

Die Erfindung wird nachfolgend anhand von Ausführungsbeispielen und den dazugehörigen Zeichnungen näher erläutert. Es zeigen:
Fig. 1 das distale Ende eines Ballonkatheters mit einer Haltestruktur;
Fig. 2a, 2b einen vergrößerten Ausschnitt aus einer Abwicklung eines Stents, wie er für eine Haltestruktur gemäß Fig. 1 nutzbar ist, und zwar im Bereich des erfindungsgemäßen Halteelementes und in Wechselwirkung mit einer erfindungsgemäßen Haltestruktur des Katheters im nicht-expandierten und im expandierten Zustand;
Fig. 3 ein Halteelement am Stent in Form von offenen Laschen und im Zusammenwirken mit einer Haltestruktur des Katheters im nicht-expandierten Zustand des Stents;
Fig. 4 das distale Ende eines Ballonkatheters mit einer weiteren, bandartigen Ausführungsform der erfindungsgemäßen Halte-struktur und

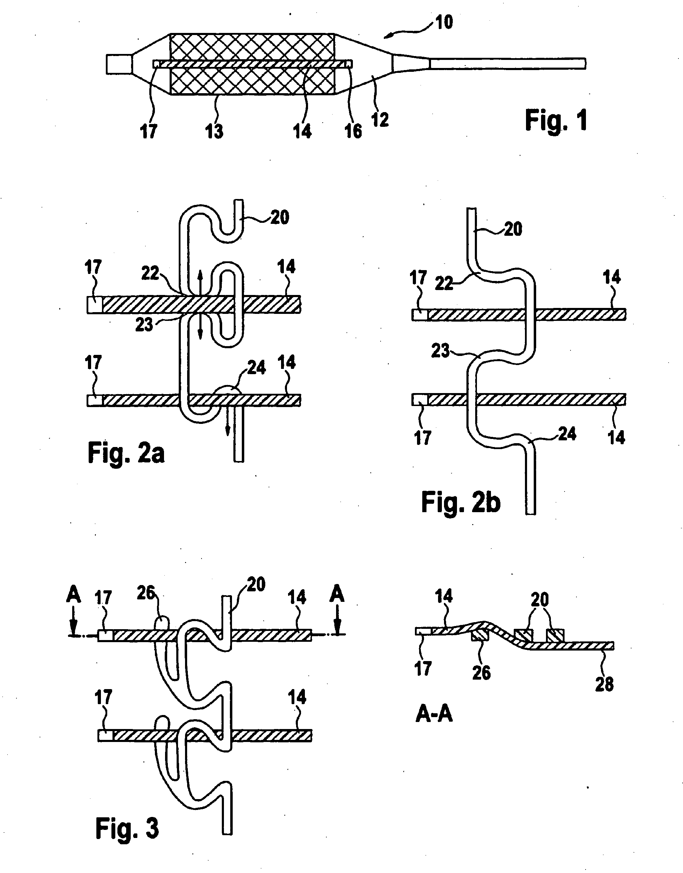

Fig. 1 ist eine vereinfachte Darstellung eines distalen Endes eines Ballonkatheters 10 mit einem dilatierbaren Ballon 12, auf den durch Crimpen ein zu Zwecken der Übersichtlichkeit nur schematisch angedeuteter Stent 13 aufgezogen wird. Der Ballon 12 wird üblicherweise aus einem thermoplastischen Polymer geformt. Nach der in Fig. 1 dargestellten Variante ist eine Haltestruktur vorgesehen, die als Band 14 ausgeführt ist, dessen beiden Enden jeweils über Verbindungsstellen 16 und 17 an den Katheter 10 gebunden sind. Die Verbindungsstellen 16 und 17 sind dem vorliegenden Beispiel zur Folge so gewählt, dass sich das Band 14 über die gesamte Länge des Ballons 12 erstreckt und das Band 14 weitgehend parallel zu einer Längsachse des Katheters 10 verläuft. Das Band 14 besteht aus einem thermoplastischen Elastomer, insbesondere einem Polyurethan, wie es auch für die Bestandteile des Katheters 10 im Bereich der Verbindungsstellen 16 und 17 Einsatz findet. Zu Zwecken der vereinfachten Darstellung ist in Fig. 1 nur ein einziges Band 14 dargestellt. Vorzugsweise weist der Katheter jedoch 3 bis 5 derartiger Bänder auf, die gleichmäßig beabstandet um den Umfang des Katheters angeordnet sind.

Die Fig. 2a und 2b sind vergrößerte Ausschnitte aus einem Applikationssystem mit einer Halterstruktur am Katheter gemäß Fig. 1 zu entnehmen. Die Figuren zeigen einen Ausschnitt aus einer Abwicklung des Stents 13, und zwar einerseits im nicht-expandierten Zustand des Stents 13 (Fig. 2a) und andererseits im expandierten Zustand des Stents 13 (Fig. 2b). Der dargestellte Ausschnitt beinhaltet eine Strebe 20, die sich ringförmig in Umlaufsrichtung fortsetzt. Der dargestellte Teil der Strebe 20 ist vorzugsweise am Stentende angeordnet, wobei aus Gründen der Übersichtlichkeit auf eine Darstellung der sich an diese ringförmig umlaufende Strebe anschließenden Strukturelemente des Stents verzichtet wurde.

Die Strebe 20 weist in dem dargestellten Ausschnitt aus Fig. 2a insgesamt 3 Abschnitte 22, 23, 24 auf, die als Halteelemente im Sinne der Erfindung agieren. Die Abschnitte 22 und 23 sind jeweils als Bogen ausgeführt, deren beide Spitzen im nicht-expandierten Zustand des Stents 13 aneinander liegen oder zumindest so nahe beieinander liegen, dass ein sich zwischen ihnen ergebener Spalt in seinen Abmessungen geringer ist, als die Breite des Bandes 14. Der bogenförmige Abschnitt 24 definiert ein weiteres Halteelement, das für die Zwecke der Erfindung im Bereich des Stents 13 bereitgestellt werden kann. Wie ersichtlich, überspannen die Bänder 14 die Abschnitte 22, 23, 24 der Strebe 20. Wird nun durch Inflation des Ballons 12 die Stentstruktur aufgeweitet, so erfährt die Strebe 20 eine Streckung bzw. die einzelnen Abschnitte 22, 23, 24 bewegen sich relativ zu den Bändern 14. Die Bewegung erfolgt derart, dass die Abschnitte 22, 23, 24 unter dem Band 14 herausgezogen werden (angedeutet durch die Pfeile in Fig. 2a). Im Ergebnis wird der Stent 13 nicht mehr über die Bänder 14 und zugehörigen Haltelemente am Stent fixiert (siehe Fig. 2b).

Fig. 3 ist eine weitere alternative Ausführungsform des Halteelementes am Stent 13 bei ansonsten gleicher Ausführungsform der Haltestruktur gemäß der vorhergehenden Figuren zu entnehmen; ebenfalls wieder beschränkt in der Darstellung auf einen Ausschnitt der Abwicklung des Stents. Eine umlaufende Strebe 20 zeigt an ihrem distalen Ende eine in Formgebung als offene Lasche bezeichenbare Struktur. Die offene Lasche wird dabei durch einen in Umlaufrichtung weisenden, an die Strebe 20 angesetzten Arm 26 gebildet. Der Fig. 3 ist ferner ein Schnitt entlang der Linie A-A zu entnehmen, der die Lage des Bandes 14 im nicht-expandierten Zustand des Stents 13 illustriert. Bei Expansion des Stents durch Balloninflation wird aufgrund der vorgegebenen Struktur der Arm 26 soweit zurückgezogen, dass er sich nicht mehr unter dem Band 14 befindet (die Bewegung wird hier nicht dargestellt).

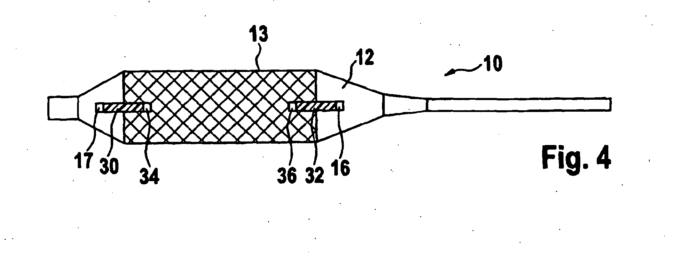

Erfindungsgemäß ist in Fig. 4 eine weitgehend an Fig. 1 angelehnte Ausführungsform des Applikationssystems zu entnehmen. Die Ausführungsform unterscheidet sich nur insoweit, als dass nicht ein den gesamten Ballon 12 überspannendes Band 14 vorgesehen ist, sondern nur zwei kurze Bänder 30, 32, deren Enden 34 bzw. 36 in dem Bereich des Ballons 12 befestigt sind, der den Stent 13 trägt oder direkt durch den Stent 13 auf dem Ballon 12 fixiert werden.

## Patentansprüche

1. Applikationssystem für einen Stent, umfassend
einen Ballonkatheter (10) mit zumindest einer mit dem Katheter fest verbundenen Haltestruktur und
einen auf den Katheter gezogenen Stent (13) mit wenigstens einem Halteelement (22, 23, 24, 26),
wobei die Haltestruktur einen Haltebereich bereitstellt, der zur Aufnahme und Festlegung des Halteelementes ausgebildet ist, und das Halteelement des Stents und die Haltestruktur des Katheters so in Geometrie und Lage aufeinander abgestimmt sind, dass
(i) sich das Halteelement im nicht-expandierten Zustand des Stents im Haltebereich der Haltestruktur befindet und
(ii) die Haltestruktur und/oder das Halteelement beim Expandieren des Stents oder zumindest im expandierten Zustand des Stents so zueinander versetzt sind, dass sich das Halteelement nicht mehr im Haltebereich der Haltestruktur befindet.
**dadurch gekennzeichnet, dass**
als Haltestruktur zwei kurze Bänder (30, 32) vorgesehen sind, deren erste Enden (34, 36) in dem Bereich des Ballons (12) befestigt sind, der den Stent (13) trägt oder direkt durch den Stent (13) auf dem Ballon (12) fixiert werden und die zweiten Enden (16, 17) der Bänder (30,32) jeweils über eine Verbindungsstelle an den Katheter (10) gebunden sind.

2. Applikationssystem nach Anspruch 1, **dadurch gekennzeichnet, dass** die Bänder (30, 32) aus einem thermoplastischen Elastomer bestehen.

3. Applikationssystem nach einem der Ansprüche 1 oder 2, **dadurch gekennzeichnet, dass** die Bänder (30, 32) und das Material des Katheters (10) im Bereich der Verbindungsstelle aus dem gleichen Material besteht.

4. Applikationssystem nach einem der Ansprüche 1 bis 3, **dadurch gekennzeichnet, dass** eine Außenkante der Bänder (30, 32) im Haltebereich zur Längsachse des Katheters (10) in einem Winkel von weniger als 45°, insbesondere weniger als 20°, besonders bevorzugt weniger als 10° steht.

5. Applikationssystem nach Anspruch 1, **dadurch gekennzeichnet, dass** das Halteelement des Stents (13) in Form einer offenen Lasche (26) ausgeführt ist.

6. Applikationssystem nach Anspruch 5, **dadurch gekennzeichnet, dass** die offene Lasche (26) am Stentende angeordnet ist.

7. Applikationssystem nach Anspruch 1, **dadurch gekennzeichnet, dass** der Stent (13) eine Strebe (20) aufweist, die im gecrimpten Zustand zumindest einen Abschnitt (22, 23, 24) mit zickzack- oder wellenförmigem Verlauf aufweist, der Abschnitt als Halteelement dient, und der Abschnitt so ausgebildet ist, dass er beim Expandieren des Stents relativ zur Haltestruktur des Katheters versetzt wird.

8. Applikationssystem nach Anspruch 7, **dadurch gekennzeichnet, dass** der Abschnitt, der als Halteelement dient, Teil einer ringförmig umlaufenden Strebe (20) des Stents (13) ist.

9. Applikationssystem nach Anspruch 8, **dadurch gekennzeichnet, dass** die ringförmig umlaufende Strebe (20) am Stentende des Stents (13) angeordnet ist

10. Katheter, umfassend ein Applikationssystem nach einem der Ansprüche 1 bis 9.

## Claims

1. An application system for a stent, comprising
a balloon catheter (10) having at least one retaining structure fixedly connected to the catheter and
a stent (13) that is pulled onto the catheter with at least one retaining element (22, 23, 24, 26),
whereby the retaining structure provides a retaining area which is designed to receive and secure the retaining element, and the retaining elements of the stent and the retaining structure of the catheter are coordinated with one another in geometry and position in such a way that
(i) in the unexpanded state of the stent, the retaining element is in the retaining area of the retaining structure and
(ii) the retaining structure and/or the retaining element is/are offset relative to one another in expansion of the stent or at least in the expanded state of the stent, so that the retaining element is no longer in the retaining area of the retaining structure,
**characterized in that**
two short bands (30, 32) are provided as the retaining structure, their first ends (34, 36) being attached in the area of the balloon (12) which carries the stent (13) or being secured directly by the stent (13) on the balloon (12), and the second ends (16, 17) of the bands (30, 32) are each bound to the catheter (10) by a connecting point.

2. The application system according to Claim 1, **characterized in that** the bands (30, 32) are made of a thermoplastic elastomer.

3. The application system according to any one of Claims 1 or 2, **characterized in that** the bands (30, 32) and the material of the catheter (10) are made of the same material in the area of the connecting point.

4. The application system according to any one of Claims 1 to 3, **characterized in that** an outer edge of the bands (30, 32) in the retaining area forms an angle to the longitudinal axis of the catheter (10) of less than 45°, in particular less than 20°, especially preferably less than 10°.

5. The application system according to Claim 1, **characterized in that** the retaining element of the stent (13) is designed in the form of an open strap (26).

6. The application system according to Claim 5, **characterized in that** the open strap (26) is arranged on the end of the stent.

7. The application system according to Claim 1, **characterized in that** the stent (13) has a strut (20) which has at least one section (22, 23, 24) with a zigzag or wavy pattern in the crimped state, said section serving as a retaining element and said section being embodied, so that when the stent expands, it is offset relative to the retaining structure of the catheter.

8. The application system according to Claim 7, **characterized in that** the section which serves as the retaining element is part of a strut (20) running around the stent (13) in the form of a ring.

9. The application system according to Claim 8, **characterized in that** the strut (20) running around the stent (13) in the form of a ring is arranged on the end of said stent.

10. The catheter comprising an application system according to any one of Claims 1 to 9.

## Revendications

1. Système d'application pour un stent, comprenant
un cathéter à ballon (10) avec au moins une structure de retenue reliée de façon fixe au cathéter et
un stent (13) étiré sur le cathéter avec au moins un élément de retenue (22, 23, 24, 26),
la structure de retenue mettant à disposition une zone de retenue qui est conçue pour le logement et la fixation de l'élément de retenue, et l'élément de retenue du stent et la structure de retenue du cathéter étant adaptés l'un à l'autre au niveau de la géométrie et de la position de telle sorte que
(i) l'élément de retenue se trouve dans la zone de retenue de la structure de retenue lorsque le stent est dans l'état non expansé et
(ii) la structure de retenue et/ou l'élément de retenue sont décalés l'un par rapport à l'autre lors de l'expansion du stent ou au moins lorsque le stent est dans l'état expansé, de telle sorte que l'élément de retenue ne se trouve plus dans la zone de retenue de la structure de retenue,
**caractérisé en ce que**
il est prévu comme structure de retenue deux bandes (30, 32) courtes, dont les premières extrémités (34, 36) sont fixées dans la zone du ballon (12) qui porte le stent (13) ou sont fixées directement par le stent (13) sur le ballon (12) et les secondes extrémités (16, 17) des bandes (30, 32) sont reliées au cathéter (10) à chaque fois par un point de liaison.

2. Système d'application selon la revendication 1, **caractérisé en ce que** les bandes (30, 32) sont sur la base d'un élastomère thermoplastique.

3. Système d'application selon l'une quelconque des revendications 1 ou 2, **caractérisé en ce que** les bandes (30, 32) et le matériau du cathéter (10) est à base du même matériau dans la zone du point de liaison.

4. Système d'application selon l'une quelconque des revendications 1 à 3, **caractérisé en ce qu'**une arête extérieure des bandes (30, 32) forme dans la zone de retenue un angle inférieur à 45°, en particulier inférieur à 20°, avec une préférence particulière inférieur à 10° par rapport à l'axe longitudinal du cathéter (10).

5. Système d'application selon la revendication 1, **caractérisé en ce que** l'élément de retenue du stent (13) est réalisé sous la forme d'un collier (26) ouvert.

6. Système d'application selon la revendication 5, **caractérisé en ce que** le collier (26) ouvert est disposé sur l'extrémité du stent.

7. Système d'application selon la revendication 1, **caractérisé en ce que** le stent (13) présente une entretoise (20) qui, dans l'état serti, présente au moins une section (22, 23, 24) avec un tracé en forme de zigzag ou ondulé, laquelle section sert d'élément de retenue, et la section est conçue de telle sorte qu'elle est décalée par rapport à la structure de retenue du cathéter lors de l'expansion du stent.

8. Système d'application selon la revendication 7, **caractérisé en ce que** la section servant d'élément de retenue fait partie d'une entretoise (20), tournant à la façon d'une bague, du stent (13).

9. Système d'application selon la revendication 8, **caractérisé en ce que** l'entretoise (20) tournant à la façon d'une bague est disposée sur l'extrémité du stent (13).

10. Cathéter, comportant un système d'application selon l'une quelconque des revendications 1 à 9.
